# EUROPÄISCHE PATENTSCHRIFT

(11) **EP 4 021 146 B1**
(45) Veröffentlichungstag und Bekanntmachung des Hinweises auf die Patenterteilung: **04.03.2026**
(21) Anmeldenummer: 21190687.0
(22) Anmeldetag: 10.08.2021
(51) Int. Cl.: H05B 6/10, A61B 50/00, A61L 2/00

(54) **INDUKTIV BEHEIZBARER ABFALLEIMER UND DOCKINGSTATION ZUR INDUKTIVEN DAMPFDEKONTAMINATION**
INDUCTIVELY HEATABLE WASTE BUCKET AND DOCKING STATION FOR INDUCTIVE STEAM DECONTAMINATION
BOÎTE À ORDURES POUVANT ÊTRE CHAUFFÉ PAR INDUCTION ET STATION D'ACCUEIL DESTINÉE À LA DÉCONTAMINATION INDUCTIVE DE LA VAPEUR

(30) Priorität: 28.12.2020 EP 20217459
(43) Veröffentlichungstag der Anmeldung: 29.06.2022
(73) Patentinhaber: Pink, Tomas, 6004 Luzern (CH)
(72) Erfinder: Pink, Tomas, 6004 Luzern (CH)
(74) Vertreter: Poljak, Michal

(56) Entgegenhaltungen:
- WO-A1-95/24228
- US-A1- 2003 230 567
- US-A1- 2011 211 989

## Beschreibung

Die vorliegende Erfindung betrifft eine Vorrichtung zur Dampfdekontamination und/oder Dampfsterilisation des Inhalts eines Behälters.

Bekannt sind Abfalleimer mit UV-Lampen aus der CN108100532A sowie ein Behältnis zur Sterilisation von gefährlichen Abfällen mittels eines hochfrequenten elektromagnetischen Feldes aus der WO 95/24228A1 .

Auch ist es unter anderem aus der US20180055595A1, der WO2016109416A1 und der WO2017129848A1 bekannt, Abfallbehälter mit Öffnung in einem Autoklaven zu positionieren und dort durch von außen eindringenden Dampf deren Inhalt zu sterilisieren.

Auch ist aus der CN204433530U und der CN104590793A ein Abfalleimer bekannt, der einen elektrischen Heizer, ein Manometer, einen Wassereinspritzanschluss, einen Wasserstandsmesser und einen Stromversorgungsanschluss aufweist.

Zudem ist ein Autoklav mit Anschlüssen für weitere Autoklavierkammern aus der EP 3 495 001 A1 bekannt.

Auch ist es aus der US 2011 211 989 A1 bekannt, metallisches Material in einem Behälter induktiv zu heizen oder, wie in der US 2003 230 567 A1 beschrieben, Flüssigkeiten in einem Metallrohr induktiv zu heizen, um sie zu verdampfen und in einen Behälter einzuleiten.

Bestehende Technologien zur effizienten Dampfsterilisation/Dampfdekontamination sind teuer, aber auch relativ sperrig und schwer, empfindlich und nicht leicht zu transportieren. Während des Transports können sie beschädigt werden oder beispielsweise die Kalibrierungsparameter der Sensoren beeinträchtigt werden, was einen großen Einfluss auf die Effizienz des Prozesses haben kann. Neben dem Elektroanschluss werden auch andere Energien und Medien gebraucht, z.B. deionisiertes Wasser, Leitungswasser, Druckluft, externe Dampfversorgung. Die Anlagen benötigen auch eine regelmäßige Wartung und den Austausch verschiedener Komponenten nach einer bestimmten Anzahl von Sterilisations/Dekontaminationszyklen.

Andere Lösungen wie die aus der CN108100532A, CN204433530U, CN104590793A bieten zudem nur unzureichende Effektivität der Sterilisation.

Die auf dem Markt erhältlichen Geräte können gefährliche Abfälle dekontaminieren, erfüllen zudem jedoch selber nicht die Hygieneanforderungen (EHEDG) und sind beispielsweise nicht für feuchte Umgebungen oder den Einsatz im Freien geeignet. Ihre Konstruktion hat keine IP-Klassifizierung und wenn sie von außen kontaminiert werden, ist es nicht wirklich möglich, sie zu dekontaminieren.

Andere, einfache Methoden, z.B. die chemische Dekontamination/Desinfektion, können nicht die hohe Effizienz bieten und verändern die chemische Zusammensetzung des Abfalls. Was zu weiteren Folgeproblemen führen kann.

Aufgabe der Erfindung ist es, eine Vorrichtung zur effizienten, einfachen und zuverlässigen Sterilisation/Dekontamination anzugeben.

Die Erfindung bietet eine technische Lösung, die es möglich macht, verschiedene Arten von Objekten, Feststoffen, Halbfeststoffen und Flüssigkeiten mit hoher Effizienz sicher und reproduzierbar zu sterilisieren/dekontaminieren, einschließlich Objekte, die bei der Prozess- und/oder Dekontaminationstemperatur durch ihren Phasenübergang gehen (schmelzen).

Das System besteht insbesondere aus zwei Teilen. Eines davon ist die Dockingstation, eine Einheit, die für viele Sterilisations-/Dekontaminationszyklen immer wieder verwendet werden kann. Diese Einheit ist an die Stromversorgung angeschlossen. Bevorzugt kommuniziert sie mit dem Benutzer (HMI), enthält Steuerelektronik und andere Elemente, die für den Betrieb des Einwegteils, des Behälters, erforderlich sind.

Der Behälter, insbesondere Abfallbehälter, selbst, der vorteilhafterweise zur Sterilisation/Dekontamination in die Dockingstation verbracht wird, wird zur Sammlung der Items verwendet und nach der Sterilisation/Dekontamination entsorgt, insbesondere verbrannt. Der Behälter weist funktionale Elemente auf, die mit der Dockingstation zusammenwirken, um die Sterilisation/Dekontamination zu gewährleisten.

Dabei wird aber nicht von außen Dampf in den Behälter überführt. Vielmehr bleibt der Behälter insbesondere geschlossen und es wird so eine Kontamination der Umgebung effizient verhindert.

Gelöst wird die Aufgabe durch einen Behälter aufweisend einen zu allen Seiten, insbesondere unter anderem durch Wandung, Boden, Zwischenboden und/oder Deckel, umschlossenen ersten Hohlraum. Dabei ist der erste Hohlraum insbesondere durch eine, insbesondere zu vier Seiten umschließende, Wandung und/oder mindestens einen, insbesondere zu einer fünften Seite verschließenden, Deckel und/oder mindestens einen, insbesondere zu einer sechsten, der fünften gegenüberliegenden Seite umschließenden, Boden und/oder Zwischenboden umschlossen. Der Behälter weist mindestens einen, insbesondere genau einen, an den ersten Hohlraum angrenzenden oder in ihm befindlichen, insbesondere unter einem Zwischenboden befindlichen, zweiten Hohlraum auf, innerhalb dessen mindestens ein erstes Heizelement angerordnet ist. Insbesondere ist der mindestens eine zweite Hohlraum in einem unteren Abschnitt des ersten Hohlraums angeordnet. Der Behälter ist erfindungsgemäß eingerichtet, den ersten und den zweiten Hohlraum oder eine Gesamtheit aus dem mindestens einen ersten und zweiten Hohlraum luftdicht gegenüber der Umgebung zu verschließen. Der Behälter ist dadurch gekennzeichnet, dass das mindestens eine erste Heizelement ein induktiv heizbares Heizelement ist. Bevorzugt ist der mindestens eine zweite Hohlraum wasser-, fluid-, gas- und/oder luftdicht verschlossen und/oder sind Mittel zum Überführen von Dampf aus dem mindesten einen zweiten Hohlraum in den mindestens einen ersten Hohlraum vorgesehen. Die Mittel zur Überführung können beispielsweise dadurch realisiert sein, dass mindestens ein, insbesondere mindestens vier, insbesondere bei und/oder durch Überdruck und/oder bei und/oder durch die Temperatur/Wärme im mindestens einen zweiten Hohlraum gegenüber dem ersten Hohlraum öffnende(n), Durchtritt(e), insbesondere in einem zwischen erstem und mindestens zweitem Hohlraum angeordneten Zwischenboden, aufweist. Der Zwischenboden kann aber auch durch eine poröse Platte, ein Gitter, Netz, Streben oder durch eine Kombination eines solchen Mittels mit einer Folie realisiert sein. Bei einem Gitter oder Netz stellen die Durchtritte (Maschen) im Gitter die Mittel zur Überführung dar.

Bevorzugt wird zum Beispiel eine nicht dampf-, wasser- und/oder luftdichte Trennung, die aber den zweiten Hohlraum vor Krafteinwirkung durch Inhalt, insbesondere Abfall, im ersten Hohlraum schützt. Dies kann beispielsweise durch ein Gitter, Streben oder ein gespanntes Netz erfolgen in Kombination mit einem wasserdichten Gefäß, das insbesondere zur Trennung eine Folie als Wandung aufweist. Das Gefäß kann beispielsweise durch einen geschlossenen Folienbeutel, in dem sich insbesondere Wasser befindet, gebildet sein. Das Gefäß ist insbesondere auf der vom ersten Hohlraum abgewandten Seite der Trennung angeordnet. Das Gefäß ist insbesondere so ausgebildet, dass es sich durch die Temperatur/Wärmeeinwirkung und/oder dadurch bewirkte Drucksteigerung öffnet, beispielsweise durch Schmelzen oder Bersten, insbesondere, insbesondere bei äußerem Normaldruck, bei einer Temperatur unter 80°C.

Als Zwischenboden können beispielsweise zwei oder ein strukturelles Element(e) dienen, beispielsweise ein Gitter und eine Folie. Grundsätzlich sollte der Zwischenboden oder zumindest eins seiner strukturellen Elemente sich unter Wärmeeinwirkung bis zumindest 140 °C nicht oder zumindest nicht wesentlich verformen und nicht schmelzen und/oder kein Wasser und keinen Wasserdampf aufnehmen und/oder gegen Wasserdampf und Wasser inert, insbesondere nicht hygroskopisch, sein.

Als Material für den Zwischenboden kommen beispielsweise in Frage Plastiknetz, Plastikgitter, Aluminiumplatte mit Löchern, Keramikplatte, poröses Graphit, Graphitfilz.

Eine solche Anordnung, bei der die Funktion des Fassens des Wassers und des Schutzes vor mechanischer Einwirkung der gesammelten Items, insbesondere des Abfalls, auf die Fassung des Wassers in getrennten Mitteln realisiert sind, kann besonders einfach und Material sparend hergestellt werden.

Das Öffnen erfolgt insbesondere durch Überdruck, insbesondere von mindestens 0,5 barg und/oder maximal 3 barg und/oder durch Temperatur/Wärme, insbesondere bei einer Temperatur im Bereich zwischen 70°C, insbesondere zwischen 80°C, insbesondere zwischen 95°C, und 140°C. Dies kann beispielsweise durch Bersten und/oder Schmelzen eines Siegels und/oder Verschlusses, beispielsweise der Folie, erfolgen. Insbesondere beginnt die Erwärmung des mindestens einen zweiten Hohlraums bei geschlossenen Mitteln zum Überführen und/oder bei vollständig geschlossenem mindestens einen zweiten Hohlraum. Insbesondere bleiben die Mittel zum Überführen und/oder der mindestens eine zweite Hohlraum vollständig geschlossen bis im mindestens einen zweiten Hohlraum eine Temperatur von mindestens 0°C und/oder ein Überdruck von mindestens 0,2 barg herrscht und/oder zumindest während des Transports und/oder während des Transports vor Einfüllung von Items, insbesondere Abfall. Der Druck wird dabei immer gegenüber dem ersten Hohlraum bestimmt.

Der zweite Hohlraum kann aber auch geöffnet sein.

In einer alternativen Ausführungsform kann das Wasser in einem dritten Hohlraum, insbesondere des Behälters, umschlossen sein und dieser vor oder durch die Erwärmung geöffnet werden und/oder ausgebildet sein durch die Erwärmung und oder dadurch bewirkten Überdruck geöffnet zu werden. Insbesondere ist der Behälter so ausgeführt, dass das Wasser aus dem dritten Hohlraum beim Öffnen des dritten Hohlraums auf und/oder um das erste induktiv heizbare Heizelement fließt. Insbesondere kann der dritte Hohlraum durch eine Tüte oder Kapsel gebildet sein, die sich im Abfallbehälter befindet.

In einer einfachen Ausführung kann aber auch einfach Wasser in den ersten Hohlraum gefüllt werden, in dem sich das erste induktiv heizbare Heizelement befindet, insbesondere bevor Abfall eingefüllt wird.

Vorteilhafterweise wird eine Dockingstation zur Aufnahme eines Behälters, insbesondere wie obenstehend beschrieben, verwendet, wobei die Dockingstation eingerichtet ist, ein elektromagnetisches Feld zum Heizen eines induktiv heizbaren Heizelements zu erzeugen und Mittel zum, insbesondere stützenden, Umschließen eines Behälters, insbesondere gegen Bersten bei Überdruck im Behälter bis zumindest 3 barg und/oder als Sicherheitsbarriere und/oder Isolation aufweist.

Ausgehend von der Erfindung kann auch ein System aufweisend mindestens eine Dockingstation und mindestens einen, insbesondere eine Vielzahl, erfindungsgemäßen/r Behälter bereitgestellt werden.

Mit Vorteil wird in dem zweiten Hohlraum Wasser mittels des ersten induktiv heizbaren Heizelements erhitzt und ein Überdruck erzeugt und Dampf in den Aufnahmeraum, insbesondere Abfallaufnahmeraum, überführt.

Bevorzugt wird also mindestens ein erstes induktiv heizbares Heizelement in einem Hohlraum, insbesondere zweiten Hohlraum, mit Wasser zur Erzeugung eines Überdrucks in dem umschlossenen Hohlraum und insbesondere zur Öffnung mindestens eines Durchtritts/en und Einbringen von Wasserdampf in einen angrenzenden Aufnahmeraum, insbesondere erster Hohlraum, zur Dampfsterilisierung des Aufnahmeraums und/oder des im Aufnahmeraum befindlichen Inhalts, verwendet.

Unter einem induktiv heizbaren Heizelement wird insbesondere ein resistiv heizendes Heizelement verstanden, das eingerichtet ist, in ihm mittels eines elektromagnetischen Feldes einen Strom insbesondere einen Ringstrom, zu induzieren, der dann zu einer resistiven Heizung führt. Das Heizelement ist also insbesondere eingerichtet, elektrischen Strom in Wärme umzuwandeln. Bevorzugt wird mittels eines elektromagnetischen Feldes ein Strom, insbesondere Ringstrom, in dem Heizelement induziert, der genutzt wird, um Wärme zu erzeugen.

Die umschließende Wandung kann beispielsweise als Zylinderwand aber auch im Querschnitt rund oder viereckig oder mehreckig ausgebildet sein. Sie kann ein-oder mehrteilig ausgeführt sein. Deckel, Boden und/oder Zwischenboden können beispielsweise fest mit der Wandung verbunden und/oder mit dieser einteilig ausgeführt sein. Mit Vorteil sind Wandung und Zwischenboden und/oder der untere Boden gemeinsam einteilig ausgeführt. Dadurch lässt sich die Stabilität erhöhen. Insbesondere ist die Wandung, der Zwischenboden, Boden und/oder Deckel doppelwandig ausgeführt. Das erhöht die Wärmedämmung.

Unter Abfall wird insbesondere biologisch kontaminierter Abfall, insbesondere solcher nach Art 3 RL 2008/98/EG verstanden. Als induktiv heizbares Heizelement kann beispielsweise ein elektrisch leitender Ring, eine elektrisch leitende Platte oder andere geeignete elektrisch leitende Struktur verwendet werden.

Unter luftdicht verschlossen ist insbesondere zu verstehen, dass der Behälter mit mechanischer Stützung von außen zumindest bis zu einem Innendruck von 1 barg, insbesondere bei 130°C, insbesondere bis zu einem Innendruck von 3 barg, insbesondere bei 130°C, luft- und/oder gasdicht ist.

Die Durchtritte sind insbesondere als Rückschlagventile ausgebildet, die bei einem Überdruck im ersten Hohlraum gegenüber dem mindestens einen zweiten Hohlraum schließen. In einer anderen bevorzugten, besonders einfachen, Ausgestaltung sind sie aus bei der Dampferzeugung im mindestens einen zweiten Hohlraum, insbesondere im Temperaturbereich von 100 bis 120 °C, schmelzendem und/oder berstendem Siegel gebildet.

Die Durchtritte sind insbesondere als Düsen ausgebildet, die insbesondere eingerichtet sind, einen gerichteten Dampfstrahl im ersten Hohlraum zu erzeugen. Dadurch lässt sich eine gute Durchmischung im ersten Hohlraum bei der Sterilisation erreichen.

Die Dockingstation kann vorteilhaft sowohl als "Stand Alone Unit" ausgebildet sein oder als "Einbaueinheit" zum Einbau in Schränke, Tisch und/oder Container und/oder in einem Schrank, Tisch und/oder Container eingebaut sein.

Unter "Single-Use"-Material wird in dieser Schrift ein Material oder eine Materialmischung verstanden, die bei einer Entsorgung, insbesondere Deponierung und/oder Verbrennung, insbesondere bei Verbrennung bei Temperaturen im Bereich von 800-1150 °C, insbesondere 800 °C und 900 °C, und/oder bei der Wirbelschichtfeuerung und/oder Rostfeuerung, keine gefährlichen Nebenprodukte oder Emissionen freigeben und entweder Rückstandslos verbrennen und/oder zerfallen oder nur Rückstände hinterlassen, die unbedenklich für Mensch und Umwelt sind und/oder solche die die Anforderungen der BlmSchG, der BImSchV und/oder Industrial Emissions Directive 2010/75/EU (Integrated Pollution Prevention and Control) in der zum Anmeldezeitpunkt letzten und/oder gültigen Fassung, insbesondere bei Verbrennung bei Temperaturen im Bereich von 800-1150 °C, insbesondere 800 °C und 900 °C, und/oder bei der Wirbelschichtfeuerung und/oder Rostfeuerung erfüllen. Bevorzugt beinhalten sie keine seltene Elemente oder anders wertvolle Materialien, insbesondere keine Materialien, deren Wert durch die Hitzeeinwirkung um mehr als 50% reduziert wird, insbesondere keine Permanentmagnete. Bevorzugt handelt es sich um Kunststoff, Emaille, Keramik, Stahl, Wolframkarbid, Titannitrid, Aluminiumoxid, Metall- und Halbmetall-Nitride, Metall-Carbide, Metall- und Halbmetall-Oxide, Glas- und/oder Kohlenstoffverbundwerkstoff, Glas- und/oder Kohlenstofffasern, Aramid, Aramidharz und/oder Aluminium und Aluminiumlegierungen, Magnesiumlegierungen, bevorzugt um Kunststoff. Als Kunststoff kommt beispielsweise in Betracht Polypropylen, Polyethylen, Polyvinylacetat, Polyethylvinylacetat, PEEK, PET, Polyamide und Polyimide.

Unter Abfall wird insbesondere biologisch kontaminierter Abfall, insbesondere solcher nach Art 3 RL 2008/98/EG, verstanden.

Mit besonderem Vorteil beinhaltet der Behälter mindestens einen Sensor, insbesondere mindestens einen Temperatur und/oder Drucksensor, wobei der mindestens eine Sensor zur drahtlosen Übertragung von Messwerten und/oder zur drahtlosen Stromspeisung, insbesondere zur Messung und/oder Übertragung von Messwerten, eingerichtet ist und/oder der mindestens eine Sensor und/oder seine Mittel zum Übertragen von Messwerten und/oder zur drahtlosen Stromspeisung in der oberen Hälfte des Behälters angeordnet sind. Mit derartigen Sensoren lässt sich der Prozess überwachen und/oder darauf basierend steuern und/oder regeln. Insbesondere sind die Übertragungsmittel zum Übertragen der Messwerte, beispielsweise mindestens eine Antenne, von den induktiv heizbaren Heizelementen entfernt, mindestens 10 cm entfernt, insbesondere in der oberen Hälfte, insbesondere in dem oberen Fünftel, des Behälters angeordnet. Oben ist insbesondere das von dem mindestens einen ersten induktiv heizbaren Heizelement entfernte und/oder dem gegenüberliegende Ende des Behälters. Dadurch lassen sich Störungen durch die Induktion vermeiden. Der mindestens eine Sensor ist mit den Mitteln zur Übertragung insbesondere mittels mindestens einem Kabel und/oder elektrischen Leiter verbunden. Mit besonderem Vorteil ist mindestens ein Druck- und/oder Temperatursensor zur Messung des Drucks und/oder der Temperatur im obersten Drittel, insbesondere im obersten Fünftel, des ersten Hohlraums vorgesehen. Bevorzugt wird mindestens ein Temperatursensor zur Messung der Temperatur im untersten Drittel, insbesondere im untersten Fünftel, des ersten Hohlraums vorgesehen. So lässt sich der Prozess besonders zuverlässig überwachen und/oder steuern und/oder regeln. Mit besonderem Vorteil ist ein Flüssigkeitsniveausensor zur Bestimmung des Flüssigkeitsniveaus im ersten Hohlraum im Behälter und/oder Dockingstation angeordnet.

Mit Vorteil ist der mindestens eine zweite Hohlraum zumindest teilweise mit Wasser gefüllt. Er ist insbesondere vor Befüllung des Behälters mit Items mit Wasser gefüllt. Das Wasser ist insbesondere vollständig im mindestens einen zweiten Hohlraum eingeschlossen. Insbesondere weist der mindestens eine zweite Hohlraum keine Öffnungen nach aussen auf. Insbesondere weist er neben den Mitteln zum Überführen keine Öffnungsmittel auf. Insbesondere sind pro 1 l Volumen des ersten Hohlraums mindestens 0,80 ml, insbesondere mindestens 2 ml, Wasser in dem mindestens einen zweiten Hohlraum vorhanden. Insbesondere sind pro 1 l Volumen des ersten Hohlraums maximal 5 ml, insbesondere maximal 100 ml, Wasser in dem mindestens einen zweiten Hohlraum vorhanden. Sind mehrere zweite Hohlräume vorhanden, sind für diese Betrachtung die Wassermengen in allen zweiten Hohlräumen zu addieren. Mit diesen Wassermengen lässt sich besonders effizient eine Dekontamination erreichen.

Mit besonderem Vorteil ist der Behälter und/oder die Wandung, der Boden, Zwischenboden und/oder der Deckel aus "Single-Use"-Material, insbesondere Kunststoff, insbesondere Polypropylen und/oder "Glass Fibre filled" Polypropylen, gebildet. Dies ist besonders Ressourcen schonend und einfach in der Herstellung.

Bevorzugt ist der Behälter so ausgebildet, dass er ohne Unterstützung von außen einem Innendruck von 3 barg bei 130°C nicht widersteht. Dies spart Konstruktionsmaterial des Behälters, was widerum besonders Ressourcen schonend ist und der Behälter ist dadurch leicht in Handhabung und Transport. Die Widerstandskraft gegen 3 barg und mehr kann durch Unterstützung, insbesondere durch die Dockingstation, erreicht werden.

Bevorzugt ist der Abfallbehälter so ausgebildet, dass er bei mechanischer Stützung und/oder Umschließung, insbesondere durch die Umschließungsmittel der Dockingstation mindestens 3 barg bei 130°C Innendruck im ersten und/oder zweiten Hohlraum widersteht. So kann eine zuverlässige Dampfsterilisation/-dekontamination ermöglicht werden.

Mit besonderem Vorteil weist der Behälter einen Druckübertragungsbereich, insbesondere eine Membran, insbesondere in der Wandung auf, der so ausgebildet ist, dass er es ermöglicht, den Innendruck im ersten und/oder zweiten Hohlraum von außen zu messen. Dazu weist die Dockingstation insbesondere einen Drucksensor zum Andrücken an diesen Druckübertragungsbereich während des Prozesses auf, um den Druck im ersten Hohlraum zu messen. Insbesondere ist sie zum Andrücken des Drucksensors, insbesondere an einen Druckübertragungsbereich ausgebildet. Dadurch lässt sich der Drucksensor mehrfach mit verschiedenen Behältern verwenden.

Bevorzugt weist der Behälter mindestens ein zweites induktiv heizbares Heizelement auf. Insbesondere ist er so ausgebildet, dass an den Wandungen des Behälters im Inneren des Behälters herabfließendes Kondensat aufgefangen und so zum mindestens einen ersten oder zweiten Heizelement geleitet wird, dass es mit dem mindestens einem ersten oder zweiten Heizelement erwärmt werden kann, insbesondere mit diesem in Kontakt gebracht wird. Dadurch lässt sich die Verdampfung von Kondensat und/oder Flüssigkeit im ersten Hohlraum zuverlässig erreichen und die Wirksamkeit der Dampfsterilisation/-dekontamination steigern.

Das erste und/oder zweite Heizelement ist/sind bevorzugt aus "Single-Use"-Material, aus Metall und/oder Metalllegierung, insbesondere ist/sind es/sie aus Aluminium oder Aluminiumlegierung gebildet und/oder ist der Behälter ausschließlich aus "Single-Use"-Material gebildet.

Mit besonderem Vorteil weist der Abfallbehälter einen verschließbaren Einwurf, insbesondere im Deckel und/oder in der Wandung auf. So ist die Befüllung einfach möglich, eine Kontaminierung der Umgebung durch Verschließen des Einwurfs aber vermeidbar und/oder reduzierbar.

Bevorzugt ist der Behälter als Abfalleimer ausgeführt. So ist die Befüllung einfach möglich.

Mit Vorteil weist der Behälter und/oder der erste Hohlraum ein Volumen im Bereich von 200 ml oder mehr und/oder von 200 l oder weniger auf. Diese Größen haben sich als besonders praktisch und geeignet herausgestellt.

Mit großem Vorteil ist der Behälter eingerichtet, so verschlossen zu werden, dass keine Öffnungen nach außen und/oder keine Durchführungen nach außen mit Ausnahme einer Abgasauslassöffnung aus dem ersten Hohlraum aus dem Behälter hinaus vorhanden sind.

Mit Vorteil weist der Behälter eine Abgasauslassöffnung vom ersten Hohlraum nach außen auf, in der sich insbesondere ein Auslassventil und/oder ein Sterilfilter, beispielweise ein Membranfilter oder ein Tiefenfilter, befindet. Durch eine solche Abgasauslassöffnung lassen sich, insbesondere nach der Dampfsterilisation/-dekontamination, gezielt und ungefährlich Überdrücke abbauen und/oder während der Dampfsterilisation/-dekontamination Abgase abführen. Durch ein Ventil lässt sich dies gezielt steuern, durch einen Filter lässt sich, vor allem zu Beginn der Dampfsterilisation/-dekontamination, das Entweichen von Kontamination verhindern.

Mit besonderem Vorteil ist die Abgasauslassöffnung mit einem flexiblen, insbesondere TPE, Schlauch mit dem ersten Hohlraum verbunden und/oder das Auslassventil als Schlauchquetschventil ausgeführt. So lässt sich besonders einfach und Ressourcen schonend ein Ventil realisieren. Auch kann die Abgasauslassöffnung mit einem Ventil, beispielsweise Membranventil, verbunden sein, wobei das Ventil insbesondere von außen, insbesondere durch Mittel der Dockingstation, mechanisch ansteuerbar und/oder mechanisch bedienbar eingerichtet ist. Insbesondere weist die Dockingstation entsprechende Mittel auf.

Bevorzugt weist der Behälter keine Kabeldurchführungen und/oder keine Stromanschlüsse, insbesondere nach außen, und/oder keine elektrischen Kontakte, insbesondere außen, auf. Dadurch lässt sich die Sicherheit erhöhen.

Mit Vorteil weist der Behälter abgesehen von einer Abgasauslassöffnung keine Verbindung von erstem und/oder zweitem Hohlraum nach außen auf, insbesondere wenn der verschließbare Einwurf geschlossen ist und/oder während der Dampfsterilisation/-dekontamination.

Mit besonderem Vorteil weist die Dockingstation Mittel zur Erzeugung eines Unterdrucks in dem Behälter, in seinem ersten Hohlraum, auf, z. B. eine Vakuumpumpe, ein Gebläse, eine Membranpumpe, eine Scroll Pumpe, eine Liquid Ring Pumpe, eine Klauenpumpe und/oder ist er ausgebildet zumindest einem Unterdruck von 0,5 barg Stand zu halten. Dadurch lassen sich Flüssigkeiten in den ersten Hohlraum saugen und/oder das Entweichen von Partikeln reduzieren.

Mit besonderem Vorteil weist der Behälter Mittel zum Anschluss von Mitteln zur Erzeugung eines Unterdrucks im ersten Hohlraum auf, z. B. eine Durchführung und/oder einen Schlauch zum Anschluss dieser Mittel. Mit besonderem Vorteil weisen die Mittel zum Anschluss einen, insbesondere hydrophoben, Sterilfilter, beispielweise einen Membranfilter oder einen Tiefenfilter auf, wobei die Mittel zum Anschluss so ausgebildet sind, dass aus dem Behälter durch die Mittel zum Anschluss abgesaugte Luft durch den Sterilfilter geleitet wird.

Mit besonderem Vorteil ist der mindestens eine zweite Hohlraum in mehrere Hohlräume unterteilt und/oder sind mehrere zweite Hohlräume vorgesehen. Insbesondere ist pro zweitem Hohlraum und/oder pro Unterteilung mindestens ein, insbesondere genau ein, Durchtritt und/oder mindestens ein zweites induktiv heizbares Heizmittel vorgesehen. Die Durchtritte sind insbesondere zwischen dem ersten Hohlraum und jedem zweiten Hohlraum und/oder jeder Unterteilung vorgesehen. Dadurch kann sichergestellt werden, dass alle Durchtritte, insbesondere wenn sie durch Überdruck im zweiten Hohlraum öffnen, öffnen. Die Unterteilung und/oder das mindestens eine zweite induktiv heizbare Heizmittel sind insbesondere so ausgebildet, dass in jeder Unterteilung ein ausreichender Druck zum Öffnen des jeweiligen Durchtritt durch Erhitzung mittels des induktiv heizbaren zweiten Heizelements aufgebaut werden kann.

Die Aufnahme in der Dockingstation kann beispielsweise durch Verrasten und/oder zumindest teilweises Umschließen erfolgen. Die Dockingstation kann zum Erzeugen des elektromagnetischen Felds zum Heizen eines induktiv heizbaren Heizelements zum Beispiel mindestens eine Induktionsspule aufweisen. Die Mittel zum, insbesondere stützenden, Umschließen eines Behälters, insbesondere gegen Bersten bei Überdruck im Behälter bis zumindest 3 barg und/oder als Sicherheitsbarriere können beispielweise durch Elemente zum formschlüssigen zumindest teilweisen Umschließen des Behälters, beispielsweise aus Metall, gebildet sein. Die können aber auch so ausgebildet sein, den Behälter nur an einer Vielzahl kleiner Bereiche mechanisch zu stützen.

Die Mittel zum Umschließen können aber auch allein als Schutz bei zerberstendem Behälter und/oder als thermische Isolation ausgebildet sein und/oder solche Elemente aufweisen. Besonders bevorzugt wird es, wenn der Behälter durch die Dockingstation vollständig umschlossen werden kann und/oder diese dazu ausgebildet ist.

Mit besonderem Vorteil sind die Mittel zum Umschließen und/oder die Dockingstation zusammenklappbar, -schiebbar und/oder -faltbar ausgebildet, sodass die Ausmaße der Dockingstation durch Zusammenklappen, - schieben und/oder -falten der Mittel zum stützenden Umschließen und/oder der Dockingstation verringert werden können, insbesondere so, dass die Ausmaße kleiner sind als der maximal aufnehmbare Behälter.

Mit Vorteil weist die Dockingstation mindestens einen Sensor, insbesondere Druck- und/oder Temperatursensor, auf, der so angeordnet ist, dass bei Aufnahme eines Behälters und Umschließen des Behälters, insbesondere mit den Mitteln zum Umschließen, der mindestens eine Sensor an den Behälter gedrückt wird. Als Sensor kann beispielsweise ein JUMO dTRANS p31 genutzt werden, dessen Edelstahlmembran insbesondere an eine flexible Plastik-Membran des Behälters angelegt und/oder angedrückt wird.

Als Sensor aus "Single-Use" Material kann beispielsweise ein PendoTECH "Single Use Pressure Sensor" verwendet werden.

Bevorzugt weist die Dockingstation Mittel zum Empfangen von drahtlos übertragenen Messdaten, insbesondere des Behälters, auf und/oder weist sie Mittel zur drahtlosen Stromspeisung von Sensoren und/oder Kommunikationsmitteln, insbesondere im Behälter, auf.

Mit Vorteil weist die Dockingstation Mittel zum Versiegeln und/oder Markieren eines aufgenommenen und/oder dekontaminierten Behälters auf. So lässt sich der Prozess besonders sicher gestalten.

Bevorzugt weist die Dockingstation Mittel zum Erkennen und/oder Auslesen einer Identifikation eines Behälters und/oder Speichern einer solchen auf. Dadurch lässt sich der Prozess, insbesondere auch durch Dokumentation, sicherer gestalten.

Mit besonderem Vorteil weist die Dockingstation mindestens einen Anschluss zum Anschluss und/oder eine Aufnahme zur Aufnahme eines Abgasauslasses eines aufgenommenen Behälters auf. Der Anschluss ist insbesondere mit einem Sterilfilter, beispielweise einem Membranfilter oder einem Tiefenfilter strömungstechnisch so verbunden, dass in den Anschluss eingeführtes Luft/Kondensat/Dampfgemisch durch den Sterilfilter geleitet wird. Dadurch lässt sich die Dampfsterilisation/-dekontamination besonders sicher gestalten. Verwendete Filter/Membranen, insbesondere hydrophobe, können zunächst Kontaminationen in Abgasen zurückhalten und zum Ende der Dampfsterilisation/-dekontamination selbst durch das durch sie geleitete Luft/Dampf/Kondensatgemisch sterilisiert/dekontaminiert werden, da das Luft-/Dampf-/Kondensatgemisch in sie eindringen kann. Insbesondere sind die Filter/Membranen entsprechend ausgebildet. Diese hydrophob auszubilden erleichtert die Sterilisation/Dekontamination, da Kondensat von ihnen abtropft oder abläuft.

Bevorzugt weist die Dockingstation, insbesondere die Aufnahme, Mittel zum Öffnen und/oder Schließen eines Abgasventils des Behälters, insbesondere des Abgasauslasses des Behälters auf, insbesondere Mittel zum Quetschen und/oder Öffnen und/oder Schließen eines Ventils eines aufgenommenen Behälters, insbesondere eines flexiblen, insbesondere TPE, Schlauchs und/oder Schlauchquetschventils des Behälters. In einer anderen Ausgestaltung kann die Aufnahme und/oder die Dockingstation ein Ventil aufweisen, wobei der Anschluss mit dem Ventil strömungstechnisch so verbunden ist, dass in den Anschluss eingeführtes Luft/Dampf/Kondensatgemisch auf das Ventil geleitet wird. Dadurch lässt sich die Dampfsterilisation/dekontamination besonders sicher gestalten und besonders zuverlässig durchführen.

Mit Vorteil ist die Dockingstation eingerichtet, oberflächendekontaminiert zu werden. Dazu weist sie insbesondere eine Ingress Protection (IP) mindestens auf dem Level 68 auf. Insbesondere weist sie keine Spalten, keine Poren und/oder keine Risse auf, in denen sich Kontamination ansammeln können. Bevorzugt weist sie keine offenen Elektrokontakte und/oder keine korrodierenden Teile auf.

Mit besonderem Vorteil erfolgt die Dampfsterilisation und/oder -dekontamination und/oder Heizung ausschließlich durch die induktiv heizbaren Heizelemente des Behälters. Insbesondere ist die Dockingstation dazu eingerichtet, die Dampfsterilisation/dekontamination des ersten Hohlraums des aufgenommenen Behälters ausschließlich mittels induktiv heizbarer Heizelemente des Behälters durchzuführen. Insbesondere weist die Dockingstation keine Heizmittel, Heizer und/oder Dampferzeuger auf. Insbesondere weist der Behälter keine anderen Heizmittel und/oder Dampferzeuger auf als die ersten und/oder zweiten induktiv heizbaren Heizmittel.

Eine mögliche Ausführungsform der Erfindung soll im Folgenden rein schematisch und nicht beschränkend anhand der folgenden Figur erläutert werden.

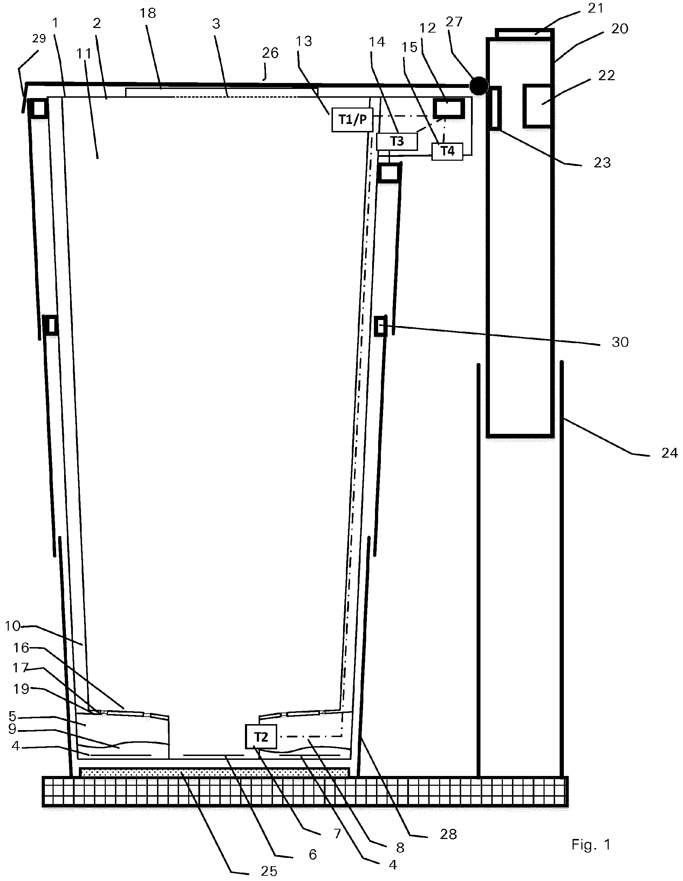

Figur 1 zeigt einen Schnitt durch eine Dockingstation 20, in der ein Behälter 1 aufgenommen ist. Zu erkennen ist ein Behälter 1, der eine doppelwandige Wandung aufweist. Die innere Wand 10 grenzt einen ersten Hohlraum 11 zu vier Seiten ab.

Nach unten hin ist er an den Seiten durch einen Zwischenboden 19 begrenzt. In der Mitte ist der Zwischenboden 19 mit dem Boden des Behälters identisch. Der Behälter weist radial angeordnet mehrere zweite Hohlräume 5 auf. Diese zweiten Hohlräume 5 sind komplett geschlossen. In ihnen ist jeweils ein erstes Heizelement 4 angeordnet, dass induktiv beheizt werden kann. Zudem ist in jedem zweiten Hohlraum 5 Wasser 9 angeordnet. Im Zwischenboden 19 sind für jeden zweiten Hohlraum 5 Ventile 17 mit Düsen in Richtung des ersten Hohlraums 11 angeordnet. Darüber hinaus verfügt der erste Hohlraum 11 an seiner tiefsten Stelle über ein induktiv heizbares zweites Heizelement 6. Zudem ist der Zwischenboden 19 so ausgebildet, dass er einen Ablauf 16 für Kondenswasser in Richtung des zweiten induktiv heizbaren Heizelement 6 darstellt.

Der Behälter 1 weist einen Einwurf 3 auf, der mittels eines Verschlusses 18 verschlossen ist.

Zudem weist der Behälter 1 mehrere Sensoren 13, 14, 15 sowie eine drahtlose Kommunikationseinrichtung 12 auf. Die drahtlose Kommunikationseinrichtung ist mit den Sensoren über elektrisch leitende Kabel verbunden. Die Sensoren beinhalten einen Temperatur und einen Drucksensor 13 zum Messen der Temperatur und des Drucks im oberen Bereich des ersten Hohlraums 11, einen Temperatursensor 15 zur Messung der Temperatur im Innenbereich der Wandung des Behälters 1, einen Temperatursensor 14 zum Messen der Temperatur der Abluftstrecke/am Sterilfilter sowie einen Temperatursensor 7 zur Messung der Temperatur im unteren Bereich des ersten Hohlraums 11. Die Dockingstation 20 weist ein Bedieninterface 21 zur Interaktion mit dem Benutzer sowie eine Kommunikationseinrichtung 22 zu Kommunikation mit anderen Bedienelementen, wie Tabletts und/oder Netzwerk auf. Die Kommunikationseinrichtung 22 ist insbesondere zur drahtlosen Kommunikation ausgestaltet.

Die Dockingstation 20 ist höhenverstellbar ausgestaltet und weist dazu eine Höhenverstellung 24 auf, mit der die Höhe der Dockingstation 20 verändert werden kann, dies insbesondere zum Verkleinern der Dockingstation zum Transport und zur Lagerung, sie kann aber auch zur Anpassung an unterschiedlich hohe Behälter 1 genutzt werden. Zudem verfügt die Dockingstation über mindestens eine Induktionsspule 25 zur induktiven Heizung der Heizelemente 4, 6. Darüber hinaus verfügt die Dockingstation über eine drahtlose Kommunikationseinrichtung 22 zur Kommunikation mit der drahtlosen Kommunikationseinrichtung 12 des Behälters 1. Zudem verfügt sie über eine als Isolationswand und Unterstützung ausgebildete Abdeckung 26, die über ein Scharnier 27 schwenkbar ist und an ihrem dem Scharnier 27 gegenüberliegenden Ende eine Verriegelung mit der höhenverstellbaren Isolationswand 28 aufweist. Die Isolationswand 28 weist zudem Unterstützungsfortsätze 30 auf, um einen aufgenommenen Behälter 1 mechanisch zu stützen und dadurch druckresistenter zu machen.

Die Dockingstation 20 ist eingerichtet, mittels der Induktionsspule 25 die induktiv heizbaren Heizelemente 4, 6 des Behälters 1 zu heizen und dadurch das Wasser 9 zum Verdampfen zu bringen und die Ventile 17 zu öffnen und Dampf in den ersten Hohlraum 11 zu überführen. Dabei werden Druck und Temperatur durch die Sensoren 13, 14, 15 gemessen und die Messwerte über die drahtlose Kommunikationseinrichtung 12 zur drahtlosen Kommunikationseinrichtung 23 übertragen. Zudem ist die Dockingstation 20 eingerichtet, mittels der drahtlosen Kommunikationseinrichtung 23 Energie zur drahtlosen Kommunikationseinrichtung 12 des Behälters 1 zu übertragen und damit die Energie für die Messungen und die Übertragung der Messwerte zu übertragen. Kondensat, dass sich zum Beispiel an der Wandung 10 des ersten Hohlraums 11 sammelt kann über den schrägen Ablauf 16 auf die zweite Induktionsheizplatte 6 geleitet und dort wieder verdampft werden.

### Bezugszeichenliste

- 1: Behälter
- 2: Deckel
- 3: Einwurföffnung
- 4: erstes induktiv heizbares Heizelement
- 5: zweiter Hohlraum
- 6: zweites induktiv heizbares Heizelement
- 7: Temperatursensor
- 8: elektrische Leitung
- 9: Wasser
- 10: innere Wand
- 11: erster Hohlraum
- 12: drahtlose Kommunikationseinrichtung
- 13: Temperatur- und Drucksensor
- 14: Temperatursensor
- 15: Temperatursensor
- 16: Ablauf
- 17: Ventil
- 18: Verschluss
- 19: Zwischenboden
- 20: Dockingstation
- 21: Bedieninterface
- 22: Kommunikationseinrichtung
- 23: drahtlose Kommunikationseinrichtung
- 24: Höhenverstellung
- 25: Induktionsspulen
- 26: Abdeckung
- 27: Scharnier
- 28: Isolationswand
- 29: Verriegelung
- 30: Unterstützungsfortsatz

## Patentansprüche

1. Ein Behälter (1) aufweisend einen, zu allen Seiten umschlossenen, ersten Hohlraum (11), wobei der Behälter (1) ein erstes Heizelement (4) aufweist, wobei das mindestens eine erste Heizelement ein induktiv heizbares Heizelement ist, **dadurch gekennzeichnet, dass** der Behälter (1) mindestens einen, an den ersten Hohlraum (11) angrenzenden oder sich im ersten Hohlraum befindlichen, zweiten Hohlraum (5) aufweist, innerhalb dessen das mindestens eine erste Heizelement (4) angeordnet ist, wobei der mindestens eine erste und der mindestens eine zweite Hohlraum (11, 5) und/oder eine Gesamtheit aus dem mindestens einen ersten und mindestens einen zweiten Hohlraum luftdicht verschlossen ausgebildet ist/sind, und **dass** Mittel zum Überführen (17) von Dampf aus dem mindestens einen zweiten Hohlraum (5) in den mindestens einen ersten Hohlraum vorgesehen sind.

2. Der Behälter nach dem vorstehenden Anspruch, wobei der Behälter (1) mindestens einen Sensor (13, 14, 15), insbesondere mindestens einen Temperatur- (14, 15) und/oder Drucksensor (13), aufweist, wobei der mindestens eine Sensor insbesondere zur drahtlosen Übertragung von Messwerten und/oder zur drahtlosen Stromspeisung eingerichtet ist und/oder der mindestens eine Sensor und/oder seine Mittel zum Übertragen von Messwerten (12) und/oder zur drahtlosen Stromspeisung in der oberen Hälfte des Behälters, angeordnet ist/sind.

3. Der Behälter nach einem der vorstehenden Ansprüche, wobei der Behälter keine Energiequelle und/oder keine Mittel zum Erzeugen eines elektromagnetischen Feldes und/oder keine elektrischen Kontakte nach außen aufweist.

4. Der Behälter nach einem der vorstehenden Ansprüche, wobei der erste und der zweite Hohlraum zumindest auch durch einen Zwischenboden (19), insbesondere mit den Mitteln zum Überführen integriert in den Zwischenboden, beispielsweise ein Gitter, Streben, ein Netz, eine Platte oder eine poröse Platte, insbesondere durch einen Zwischenboden (19) oder einen Zwischenboden (19) und eine Folie, voneinander getrennt sind.

5. Der Behälter nach einem der vorstehenden Ansprüche, wobei der mindestens eine zweite Hohlraum zumindest teilweise mit Wasser (9) gefüllt ist und/oder der Behälter, insbesondere als zweiten Hohlraum oder als Teil des zweiten Hohlraums, ein wasserdichtes Gefäß, insbesondere Beutel oder Kapsel, das insbesondere eine Folie als Wandung aufweist, welches mit Wasser gefüllt ist, aufweist, wobei das Gefäß insbesondere auf der vom ersten Hohlraum abgewandten Seite einer Trennung angeordnet ist und/oder wobei das Gefäß insbesondere so ausgebildet ist, dass es sich durch Temperatur und/oder Wärmeeinwirkung und/oder dadurch bewirkte Drucksteigerung öffnet, insbesondere durch Schmelzen oder Bersten bei einer Temperatur unter 80 °C und/oder über 50 °C.

6. Der Behälter nach einem der vorstehenden Ansprüche, wobei der Behälter mindestens ein zweites induktiv heizbares Heizelement (6) aufweist und/oder so ausgebildet ist, dass an den Wandungen des Behälters im Inneren des Behälters herabfließendes Kondensat, aufgefangen wird und/oder so zum mindestens einen ersten oder mindestens einen zweiten Heizelement geleitet wird, dass es mit dem mindestens einen ersten oder mindestens einen zweiten Heizelement erwärmt werden kann, insbesondere mit diesem in Kontakt gebracht wird.

7. Der Behälter nach einem der vorstehenden Ansprüche, wobei der Behälter eingerichtet ist, so verschlossen zu werden, dass keine Öffnungen nach Außen und/oder keine Durchführungen nach Außen mit Ausnahme einer Auslassöffnung aus dem ersten Hohlraum aus dem Behälter hinaus vorhanden sind.

8. Der Behälter nach einem der vorstehenden Ansprüche, wobei er eine Abgasauslassöffnung vom ersten Hohlraum nach außen aufweist, in dem insbesondere ein Auslassventil und/oder ein Sterilfilter, beispielweise Membranfilter oder Tiefenfilter sich befindet und/oder wobei die Abgasauslassöffnung mit einem flexiblen, insbesondere TPE, Schlauch mit dem ersten Hohlraum verbunden ist und/oder das Auslassventil als Schlauchquetschventil ausgeführt ist.

9. Der Behälter nach einem der vorstehenden Ansprüche, wobei der mindestens eine zweite Hohlraum in mehrere Hohlräume unterteilt ist und/oder pro zweitem Hohlraum und/oder Unterteilung ein Durchtritt vorgesehen ist, insbesondere zwischen dem ersten Hohlraum und jedem zweiten Hohlraum und/oder jeder Unterteilung.

## Claims

1. A container (1) comprising a first cavity (11) enclosed on all sides, wherein the container (1) comprises a first heating element (4), wherein the at least one first heating element is an inductively heatable heating element, **characterized in that** the container (1) comprises at least one second cavity (5) which adjoins the first cavity (11) or is located in the first cavity, and within which second cavity (5) the at least one first heating element (4) is arranged, wherein the at least one first and the at least one second cavity (11, 5) and/or an entirety of the at least one first and the at least one second cavity is/are constructed in an airtight manner, and **in that** means for transferring (17) steam from the at least one second cavity (5) into the at least one first cavity are provided.

2. The container (1) according to the preceding claim, wherein the container (1) comprises at least one sensor (13, 14, 15), in particular at least one temperature sensor (14, 15) and/or pressure sensor (13), wherein the at least one sensor is configured in particular for wireless transmission of measured values and/or for wireless power supply and/or the at least one sensor and/or means thereof for transmitting measured values (12) and/or for wireless power supply is/are arranged in the upper half of the container.

3. The container according to one of the preceding claims, wherein the container comprises no power source and/or no means for generating an electromagnetic field and/or no electrical contacts to the outside.

4. The container according to any one of the preceding claims, wherein the first and second cavity are separated from one another also by at least an intermediate base (19), in particular with the means for transferring integrated in the intermediate base, for example a grid, struts, a net, a plate or a porous plate, in particular by an intermediate base (19) or by an intermediate base (19) and a film.

5. The container according to any one of the preceding claims, wherein the at least one second cavity is at least partially filled with water (9) and/or the container comprises, in particular as the second cavity or as part of the second cavity, a water-tight vessel, in particular a pouch or a capsule, which in particular comprises a film as a wall, which water-tight vessel is filled with water, wherein the vessel is in particular arranged on the separation side facing away from the first cavity and/or wherein the vessel is in particular constructed such that it opens by temperature and/or heat action and/or pressure increase caused thereby, in particular by melting or bursting at a temperature below 80 °C and/or above 50 °C.

6. The container according to any one of the preceding claims, wherein the container comprises at least one second inductively heatable heating element (6) and/or is constructed in such a way that a condensate flowing down on the walls of the container inside the container is collected and/or conducted to the at least one first or at least one second heating element in such a way that it can be heated with the at least one first or at least one second heating element, in particular by being brought into contact therewith.

7. The container according to any one of the preceding claims, wherein the container is configured to be closed such that no openings are provided to the outside and/or no passages are provided to the outside except for an outlet opening out of the first cavity out of the container.

8. The container according to one of the preceding claims, wherein it comprises an exhaust gas outlet opening from the first cavity to the outside, in which in particular an outlet valve and/or a sterile filter, for example a membrane filter or a depth filter, is located and/or wherein the exhaust gas outlet opening is connected with a flexible, in particular TPE, hose to the first cavity and/or the outlet valve is constructed as a hose pinch valve.

9. The container according to any one of the preceding claims, wherein the at least one second cavity is partitioned into a plurality of cavities and/or a passage is provided per second cavity and/or per partition, in particular between the first cavity and each second cavity and/or each partition.

## Revendications

1. Un récipient (1) contenant une première cavité (11) enfermée sur tous les côtés, où le récipient (1) contient un premier élément chauffant (4), où l'au moins un premier élément chauffant est un élément chauffant chauffable par induction, **caractérisé en ce que** le récipient (1) contient au moins une seconde cavité (5) qui est contiguë à la première cavité (11) ou est située dans la première cavité et à l'intérieur de laquelle l'au moins un premier élément chauffant (4) est disposé, où l'au moins une première et l'au moins une seconde cavité (11, 5) et/ou une totalité de l'au moins une première et l'au moins une seconde cavité est/sont prévue(s) de manière étanche à l'air, et **en ce que** des moyens pour transférer (17) de la vapeur de l'au moins une seconde cavité (5) dans l'au moins une première cavité sont prévus.

2. Le récipient (1) selon la revendication précédente, où le récipient (1) contient au moins un capteur (13, 14, 15), en particulier au moins un capteur de température (14, 15) et/ou un capteur de pression (13), où l'au moins un capteur est configuré en particulier pour la transmission sans fil de valeurs mesurées et/ou pour l'alimentation électrique sans fil et/ou l'au moins un capteur et/ou ses moyens pour transmettre des valeurs mesurées (12) et/ou pour une alimentation électrique sans fil dans la moitié supérieure du récipient est/sont disposé(s).

3. Le récipient selon l'une des revendications précédentes, où le récipient ne contient aucune source d'énergie et/ou aucun moyen pour générer un champ électromagnétique et/ou aucun contact électrique vers l'extérieur.

4. Le récipient selon l'une quelconque des revendications précédentes, où la première et la seconde cavité sont séparées l'une de l'autre au moins également par une base intermédiaire (19), en particulier avec les moyens pour transférer, intégrés dans la base intermédiaire, par exemple une grille, des entretoises, un filet, une plaque ou une plaque poreuse, en particulier par une base intermédiaire (19) ou une base intermédiaire (19) et un film.

5. Le récipient selon l'une quelconque des revendications précédentes, où l'au moins une seconde cavité est au moins partiellement remplie d'eau (9) et/ou le récipient, en particulier en tant que seconde cavité ou en tant que partie de la seconde cavité, contient un récipient étanche à l'eau, en particulier un sac ou une capsule, qui en particulier contient un film en tant que paroi, où le récipient étanche à l'eau est rempli d'eau, où le récipient est en particulier disposé sur le côté d'une séparation opposée à la première cavité et/ou où le récipient est en particulier conçu de telle sorte qu'il s'ouvre par une action de température et/ou de chaleur et/ou une augmentation de pression provoquée par celui-ci, en particulier par fusion ou éclatement à une température inférieure à 80 °C et/ou supérieure à 50 °C.

6. Le récipient selon l'une quelconque des revendications précédentes, où le récipient contient au moins un second élément chauffant chauffable par induction (6) et/ou est conçu de telle sorte qu'un condensat s'écoulant vers le bas sur les parois du récipient à l'intérieur du récipient est collecté et/ou conduit vers l'au moins un premier ou l'au moins un second élément chauffant de telle manière qu'il peut être chauffé avec l'au moins un premier ou l'au moins un second élément chauffant, en particulier qu'il est mis en contact avec celui-ci.

7. Le récipient selon l'une quelconque des revendications précédentes, où le récipient est configuré pour être fermé de sorte qu'aucune ouverture vers l'extérieur et/ou aucun passage vers l'extérieur n'est prévu(e) à l'exception d'une ouverture de sortie hors de la première cavité hors du récipient.

8. Le récipient selon l'une des revendications précédentes, où il contient une ouverture de sortie de gaz d'échappement de la première cavité vers l'extérieur, où en particulier une soupape de sortie et/ou un filtre stérile, par exemple un filtre à membrane ou un filtre de profondeur, est situé et/ou où l'ouverture de sortie de gaz d'échappement est reliée par un tuyau flexible, en particulier TPE, à la première cavité et/ou la soupape de sortie est conçue sous la forme d'une vanne à pincement de tuyau.

9. Le récipient selon l'une quelconque des revendications précédentes, où l'au moins une seconde cavité est divisée en une pluralité de cavités et/ou un passage est prévu par seconde cavité et/ou par partition, en particulier entre la première cavité et chaque seconde cavité et/ou chaque partition.
